# EUROPÄISCHE PATENTSCHRIFT

(11) **EP 1 368 331 B1**
(45) Veröffentlichungstag und Bekanntmachung des Hinweises auf die Patenterteilung: **09.04.2014**
(21) Anmeldenummer: 02719875.3
(22) Anmeldetag: 20.02.2002
(51) Int. Cl.: C07D 309/32

(54) **KONTINUIERLICHES VERFAHREN ZUR HERSTELLUNG VON DIHYDROPYRONEN**
CONTINUOUS METHOD FOR PRODUCING DIHYDROPYRONES
PROCEDE CONTINU DE PRODUCTION DE DIHYDROPYRONES

(30) Priorität: 22.02.2001 DE 10108471
(43) Veröffentlichungstag der Anmeldung: 10.12.2003
(73) Patentinhaber: Boehringer Ingelheim Pharma GmbH & Co. KG, 55216 Ingelheim am Rhein (DE)
(72) Erfinder: SAUTER, Markus, 55457 Gensingen (DE); MEYER, Oliver, 55452 Dorsheim (DE); GÖHLICH, Mark, 55437 Appenheim (DE)
(86) Internationale Anmeldenummer: PCT/EP2002/001772
(87) Internationale Veröffentlichungsnummer: WO 2002/068403

(56) Entgegenhaltungen:
- WO-A-00/15625
- WO-A-01/09064
- WO-A-01/51434
- WO-A-95/14012
- WO-A-98/19997
- WO-A-98/37457
- TURNER,S.R. ET AL.: "Tipranavir (PNU-146690) : A Potent , Orally Bioavailable Nonpeptidic HIV Protease Inhibitor of the 5,6-Dihydro-4-hydroxy- 2-pyrone Sulfonamide Class" J.MED.CHEM., Bd. 41, Nr. 18, 1998, Seiten 3467-3476, XP002200332 WASHINGTON in der Anmeldung erwähnt
- AUTORENKOLLEKTIV: "Organikum" 1976 , VEB DEUTSCHER VERLAG DER WIISENSCHAFTEN , BERLIN XP002200333 Seite 579 -Seite 589
- KAMPER K-P ET AL: "Microfluidic components for biological and chemical microreactors" MICRO ELECTRO MECHANICAL SYSTEMS, 1997. MEMS ' 97, PROCEEDINGS, IEEE., TENTH ANNUAL INTERNATIONAL WORKSHOP ON NAGOYA, JAPAN 26-30 JAN. 1997, NEW YORK, NY, USA,IEEE, US, 26. Januar 1997 (1997-01-26), Seiten 338-343, XP010216929 ISBN: 0-7803-3744-1
- "MICROREACTIORS FIND NEW NICHES" CHEMICAL ENGINEERING, MCGRAW-HILL. NEW YORK, US, 1. März 1997 (1997-03-01), Seiten 30-31,33, XP000197691 ISSN: 0009-2460
- EHRFELD W ET AL: "POTENTIALS AND REALIZATION OF MICROREACTORS" DECHEMA MONOGRAPHIEN, VERLAG CHEMIE, WEINHEIM,, DE, Bd. 132, 1995, Seiten 1-28, XP000925630 ISSN: 0070-315X

## Beschreibung

Die Erfindung betrifft ein verbessertes Verfahren zur Herstellung von Dihydropyronen der allgemeinen Formel I, 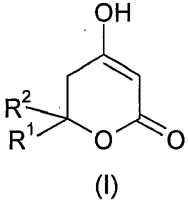 worin die Reste R¹ und R² die in den Ansprüchen genannten Bedeutungen haben können.

### Hintergrund der Erfindung

Dihydropyrone sind als Zwischenprodukte in der Arzneimittelsynthese von Bedeutung. Insbesondere ist 5,6-Dihydro-4-hydroxy-6-phenethyl-6-propyl-2H-pyran-2-on ein wichtiges Zwischenprodukt in der Synthese von Tipranavir, einem HIV Protease Inhibitor. Die Verbindungen der Formel I sowie Verfahren zu deren Herstellung sind aus dem Stand der Technik bekannt.

In der WO 98/19997 werden verschiedene Methoden zur Herstellung der Dihydropyron-Zwischenstufen beschrieben, basierend auf der Herstellung einer Keton-Vorstufe und anschließender Zyklisierung des Ketons in das gewünschte Dihydropyron-Ringsystem.

In Journal of Medicinal Chemistry, 1998, Vol. 41, No. 18, 3467 wird ein Verfahren zur Herstellung eines racemischen Gemisches von 5,6-Dihydro-4-hydroxy-6-phenethyl-6-propyl-2H-pyran-2-on beschrieben, welches in einem Schritt a) die Reaktion eines Dianions des Methylacetoessigesters mit 1-Phenyl-3-hexanon und in einem anschließenden Schritt b) die Zyklisierung des entstanden-en β-Ketoesters mittels alkalischer Hydrolyse und darauffolgender Acidifizierung beinhaltet. Dieses Verfahren wird diskontinuierlich durchgeführt und erzielt eine Ausbeute von 72 %.

In Organikum, VEB Deutscher Verlag der Wissenschaften, Berlin 1976, 579-589, sind die Grundlagen von Esterkondensationen vom Aldoltyp beschrieben.

In der WO 00/15625 werden Verfahren zur Herstellung von Dihydropyron HIV Protease Inhibitoren unter Verwendung Aldol-artiger Kondensationsreaktionen beschrieben.

In der WO 95/14012 werden 5,6-Dihydropyrone als HIV Protease Inhibitoren sowie Methoden zu deren Synthese ebenfalls unter Verwendung Aldol-artiger Kondensationsreaktionen und Zyklisierung zum Dihydropyron-Ringsystem beschrieben.

In der WO 98/37457 werden chemische Mikroreaktoren, deren Herstellung und diverse Einsatzmöglichkeiten für diese Reaktoren beschrieben.

In der WO 01/09064 wird die Durchführung von Friedel-Crafts Acylierungen organischer Verbindungen in temperierbaren Mikroreaktoren beschrieben.

In Micro Electro Mechanical Systems, 1997, MEMS' 97, Proceedings, IEEE., US, 338-343 (ISBN: 0-7803-3744-1) wird ein kurzer Überblick über miniaturisierte Reaktionssysteme zusammengefaßt.

Weitere miniaturisierte Reaktionssysteme und einige Verwendungsmöglichkeiten derartiger Systeme sind in Chemical Engineering, McGraw-Hill, New York, US, 1997, 30-31, 33 (ISSN: 0009-2460) beschrieben.

In Dechema Monographien, Verlag Chemie, Weinheim, DE, Vol. 132, 1995, 1-28, wird der damalige Stand der Technik zur Herstellung 3-dimensionaler Mikrostrukturen sowie die bestehende Mikroreaktor-Technologie zusammengefaßt.

In der WO 01/51434 werden Verfahren zur Durchführung von Reaktionen von Carbonylverbindungen mit metallorganischen Reagenzien, insbesondere mit Grignardverbindungen, beschrieben, in denen das metallorganische Reagenz und die Carbonylverbindung jeweils in einem geeigneten Lösungsmittel getrennt vorgelegt und temperiert werden und danach zur Reaktion in einen temperierten Mini/Mikromischer gepumpt werden.

Es ist daher die Aufgabe der Erfindung ein Verfahren bereitzustellen, welches die Herstellung von Dihydropyranonen in hoher Reinheit und eine signifikante Verbesserung der Ausbeute im Vergleich zum Stand der Technik ermöglicht.

### Detaillierte Beschreibung der Erfindung

Überraschenderweise wurde gefunden, dass die Verbindungen der Formel I in hoher Reinheit und in wesentlich höherer Ausbeute erhalten werden können, wenn Schritt a) kontinuierlich in einem Mikroreaktor durchgeführt wird.

Die Erfindung betrifft daher ein im Labormaßstab und ebenso technisch anwendbares Verfahren zur Herstellung einer Verbindung der allgemeinen Formel (I), 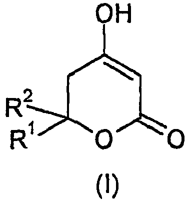 worin,
R¹ für einen C₁-C₈-Alkyl-, C₆-C₁₀-Aryl-C₁-C₄-alkyl-, oder C₃-C₈-Cycloalkyl-C₁-C₄-alkyl- Rest, und
R² für einen C₁-C₈-Alkyl- Rest steht,
durch Umsetzung
a) eines Ketons der Formel (II) 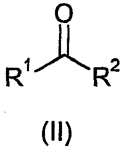 worin R¹ und R² die vorstehend genannte Bedeutung aufweisen,
   in Gegenwart einer starken Base mit einem Acetessigester
   und
durch Zyklisierung b) der erhaltenen Verbindung der Formel (IV) 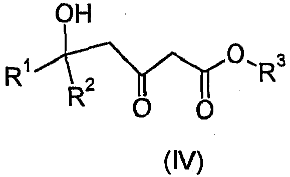 worin,
   R³ für einen C₁-C₄-Alkyl oder Benzylrest steht,
   wobei das Keton der Formel II kontinuierlich mit einem Acetessigester in Form seines Dianions
   in einem Mikroreaktor umgesetzt wird.

Die für das erfindungsgemäße Verfahren geeigneten Mikroreaktoren sind beispielsweise aus "Microreactors; Wolfgang Ehrfeld, Volker Hessel, Holger Löwe; Wiley-VCH;ISBN 3-527-29590-9; Kapitel 3 Micromixers" bekannt. In dem erfindungsgemäßen Verfahren einsetzbare Mikroreaktoren besitzen in der Regel ein Gehäuse aus Edelstahl, Glas,Titan oder Metall-Legierungen und ein Inlay bzw. Inlay-Strukturen aus thermisch oxidiertem Silizium, Kupfer, Aluminium, Nickel, Silber, Metall-Legierungen, Foturan-Glas oder metallbeschichteten Kunststoff-, Glas- oder Keramikwerkstoffen.

Die Mischung der Eduktströme kann sowohl turbulent als auch laminar, bevorzugt laminar erfolgen. Zu den für eine laminare Mischung bevorzugten Kanalstrukturen gehören in der Regel interdigitale Strukturen, sternenförmige Strukturen oder Strukturen eines Raupenmischers. Für das erfindungsgemäße Verfahren einsetzbare Mikroreaktortypen sind beispielsweise von den Firmen Institut für Mikrotechnik Mainz GmbH, Cellular Process Chemistry GmbH oder Mikroglas AG erhältlich.

Erfindungsgemäß besonders bevorzugt ist ein Verfahren, in dem für Reaktionsschritt a) ein Mikroreaktor mit interdigitaler Kanalstruktur, insbesondere bevorzugt ein Mikroreaktor vom Typ LIGA (Herstellungsverfahren mittels Lithographie, Galvanoformung, Abformung) mit interdigitaler Kanalstruktur, beispielsweise hergestellt vom Institut für Mikrotechnik Mainz GmbH, verwendet wird.

Besonders bevorzugt ist ein Verfahren, in dem ein die Verbindung der Formel (II) enthaltender Eduktstrom A und ein einen Acetessigester in Form eines Dianions enthaltender Eduktstrom B kontinuierlich in dem Mischelement eines Mikroreaktors miteinander vermischt werden und das flüssige Reaktionsgemisch in eine Kapillare, insbesondere eine Verweilkapillare, geführt wird.

Ferner besonders bevorzugt ist ein Verfahren, in dem die Kapillare eine Länge von 0,1 bis 10 m, vorzugsweise 0,3 bis 8 m, bevorzugt 0,5 bis 6 m, besonders bevorzugt 0,8 bis 4 m, insbesondere bevorzugt etwa 1 m und einen Innendurchmesser von 0,05 bis 5 mm, vorzugsweise 0,1 bis 4 mm, bevorzugt 0,3 bis 3 mm, insbesondere bevorzugt etwa 1 mm besitzt.

Insbesondere bevorzugt ist ein Verfahren, wobei in Schritt a) als Verbindung der Formel (II) 1-Phenyl-3-hexanon eingesetzt wird.

Ferner insbesondere bevorzugt ist ein Verfahren, wobei in Schritt a) der Acetessigester als Dilithium, Monolithiummononatrium- oder Dinatriumsalz eingesetzt wird.

Von besonderer Bedeutung ist ein Verfahren, wobei die Verbindung der Formel (III) zu der Verbindung der Formel (II) in einem molaren Verhältnis von 2:1 bis 1:2, bevorzugt 1:1 bis 1:1,5, insbesondere bevorzugt 1:1 bis 1:1,2, ganz besonders bevorzugt von etwa 1:1 1 eingesetzt wird.

Ferner von besonderer Bedeutung ist ein Verfahren, wobei die Reaktion in Schritt a) bei einer Temperatur von -78 bis +85 °C, vorzugsweise bei -40 bis +50 °C, bevorzugt bei -30 bis +20 °C, besonders bevorzugt bei -25 bis +10 °C, insbesondere bevorzugt bei -20 bis 0 °C, ganz besonders bevorzugt bei -15 bis -5 °C, überaus bevorzugt bei etwa -10 °C durchgeführt wird.

Weiterhin bevorzugt ist ein Verfahren, wobei die Reaktion in Schritt a) bei einer Gesamtflussrate, welche durch Addition der Flussraten der Verbindung der Formel II und des Acetessigesters errechnet wird, von 1,5 bis 5 ml/min, bevorzugt bei 1,8 bis 4 ml/min, besonders bevorzugt bei 2 bis 3,5 ml/min, insbesondere bevorzugt bei etwa 2,5 ml/min, durchgeführt wird.

Weiterhin besonders bevorzugt ist ein Verfahren, wobei die Flussrate der Verbindung der Formel (II) zu der des Acetessigesters in einem Verhältnis von 1:1 bis 1:2, bevorzugt von 1:1,1 bis 1:1,8, besonders bevorzugt von 1:1,2 bis 1:1,5, insbesondere bevorzugt von etwa 1:1,3 steht.

Zur Erzielung der Flussraten ist in der Regel der Einsatz pulsationsarmer Pumpen, vorzugsweise Drehkolbenpumpen, bevorzugt Keramikdrehkolben-Pumpen oder HPLC- Pumpen von Vorteil. Die Flussraten können im Sinne einer optimalen Raum-/Zeit-Ausbeute verschiedenen Reaktortypen angepaßt werden.

Weiterhin insbesondere bevorzugt ist ein Verfahren, wobei die Reaktion in mehreren seriell oder parallel geschalteteten Mikroreaktoren durchgeführt wird.

Bevorzugt ist die Verwendung des nach dem erfindungsgemäßen Verfahren erhaltenen 5,6-Dihydro-4-hydroxy-6-phenethyl-6-propyl-2H-pyran-2-on zur Herstellung von Tipranavir.

In der vorliegenden Erfindung steht der Begriff "Alkyl" für eine geradkettige oder verzweigte Alkylgruppe mit 1 bis 8 C-Atomen, vorzugsweise 2 bis 7 C-Atomen, bevorzugt 3 bis 6 C-Atomen. Insbesondere bevorzugt sind Methyl, Ethyl, n-Propyl, iso-Propyl, n-Butyl, iso-Butyl, sec. Butyl, tert.-Butyl, n-Pentyl, iso-Pentyl oder neo-Pentyl.

Der Begriff "Aryl" steht für einen aromatischen Kohlenwasserstoffrest mit 6 bis 10 C-Atomen, vorzugsweise Phenyl oder Naphthyl, besonders bevorzugt Phenyl, welches mit einer oder mehreren Alkylgruppen substituiert sein kann.

Als Cycloalkylreste mit 3 - 8 Kohlenstoffatomen werden Cyclopropyl, Cyclobutyl, Cyclopentyl, Cyclohexyl, Cycloheptyl oder Cyclooctyl bezeichnet.
R¹ ist vorzugsweise Phenylmethyl, Phenylethyl oder Phenylpropyl, insbesondere bevorzugt 2-Phenylethyl,
R² ist vorzugsweise Methyl, Ethyl, n-Propyl oder n-Butyl, insbesondere bevorzugt n-Propyl.
R³ ist vorzugsweise Methyl, Ethyl, n-Propyl oder Benzyl, insbesondere bevorzugt Ethyl.

Als starke Basen werden vorzugsweise Metallhydride, Metallorganyle, Metallamide, Metalldialkylamide oder Metallhexamethyldisilazane eingesetzt.

Als Metallkationen werden beispielsweise Lithium-, Natrium-, Kalium-, Rubidium-, Cäsium-, Magnesium-, Calcium-, Titan-, Silicium, Zinn- und Lanthanoide , vorzugsweise Lithium oder Natrium, besonders bevorzugt Lithium bezeichnet.

Ganz besonders bevorzugte Basen sind Natriumhydrid, Lithiumdiethylamid, Butyllithium, Lithiumdiisopropylamid, Lithiumhexamethyldisilazan, Natriumhexamethyldisilazan oder Kaliumhexamethyldisilazan oder Kombinationen aus diesen. In der Regel werden 2 oder mehr Equivalente dieser Basen eingesetzt, vorzugsweise 1,8 bis 3,0, insbesondere 1,9 bis 2,5 Equivalente. Der Acetessigester liegt in Gegenwart dieser Basen in der Regel in Form eines Dianions der Formel III vor.

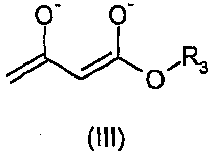

Das erfindungsgemäße Verfahren wird in der Regel in Gegenwart eines inerten Verdünnungsmittels durchgeführt. Bevorzugte Verdünnungsmittel sind unpolare organische Lösungsmittel wie z.B. aliphatische oder aromatische Kohlenwasserstoffe, Ether oder Gemische derselben. In einer besonders bevorzugten Ausführungsform wird das Verdünnungsmittel ausgewählt aus der Gruppe bestehend aus Dimethoxyethan, Diethylether, *tert*-Butyl-methylether, Tetrahydrofuran, n-Hexan, Cyclohexan, Toluol, Xylol oder einem Gemisch dieser Lösungsmittel, insbesondere Tetrahydrofuran und Dimethoxyethan.

Neben den genannten Verdünnungsmitteln kann das Reaktionsgemisch auch ein oder mehrere Amine wie zum Beispiel Diethylamin, Diisopropylamin oder Tetramethylethylendiamin enthalten.

Der Vorteil des erfindungsgemäßen Verfahrens liegt in der hohen Reinheit und der unerwartet hohen Ausbeute an Dihydropyranon von über 90 %, welche aus dem kontinuierlichen Mikroreaktorverfahren des Schritt a) resultiert. Die Verbindung der Formel IV kann als Produkt des Mikroreaktorverfahrens ohne weitere Aufreinigung weiterverarbeitet werden.

Die folgenden Beispiele dienen der näheren Erläuterung des erfindungsgemäßen Verfahrens. Sie sind lediglich als exemplarische Vorgehensweise zu verstehen, ohne die Erfindung auf deren Inhalt zu beschränken.

### Beispiel 1: 5,6-Dihydro-4-hydroxy-6-phenethyl-6-propyl-2H-pyran-2-on

### Schritt a)

In einen Mikroreaktor der Firma Institut für Mikrotechnik Mainz GmbH (Typ Liga mit interdigitaler Kanalstruktur) werden ein Gemisch I aus 81,9 g 1-Phenyl-3-hexanon und 840 ml Tetrahydrofuran und ein Gemisch II aus 72,9 g Ethyl-Acetessigester, 117 ml Diethylamin und 450 ml n-Butyllithium in n-Hexan (2,5 molar) in 361 ml Tetrahydrofuran bei -10 °C gegeneinander gepumpt und gemischt. Dabei wird der Volumenstrom des Gemisches I bei 1 ml/min und der Volumenstrom des Gemisches II bei 1,1 ml/min eingestellt. Die Produktlösung wird durch eine Kapillare (Länge 1 m, Durchmesser 1 mm) geleitet und anschließend in gesättigter Ammoniumchlorid-lösung/Salzsäure-Lösung bei einem pH-Wert von 5 - 6 aufgefangen.

### Schritt b)

140 g des aus Schritt a) resultierenden rohen ß-Ketoesters werden bei 5 bis 10 °C in 200 ml Methanol aufgenommen. Unter Rühren wird bei 5 bis 10 °C festes Kaliumhydroxid zugegeben und anschließend etwa 15 Stunden bei Raumtemperatur gerührt. Das Methanol wird abdestilliert und der Rückstand mit 500 ml Wasser versetzt. Es wird zweimal mit je 200 ml Toluol extrahiert. Nach Abtrennung der organischen Phase wird erneut 400 ml frisches Toluol zur wässrigen Phase gegeben. Diese wird mit konz. Schwefelsäure bis zu pH 1,9 angesäuert. Die wässrige Phase wird abgetrennt und die organische Phase noch 3 mal mit Wasser ausgeschüttelt. Die organische Phase wird im Vakuum (60 mbar) bei 40 °C zur Trockne eingedampft. Das Rohprodukt wird in 200 ml Toluol bei 60 °C gelöst und anschließend filtriert. Zu dem Filtrat wird unter Rühren langsam 200 ml n-Octan bei 40 °C zugetropft. Es wird mit 5,6-Dihydro-4-hydroxy-6-phenethyl-6-propyl-2H-pyran-2-on Kristallen geimpft und etwa 15 Stunden bei Raumtemperatur gerührt. Zu der entstandenen Kristallmasse werden 400 ml n-Octan getropft und auf 0 - 5 °C abgekühlt. Nach etwa 1 stündigem Nachrühren bei 0 - 5 °C werden die Kristalle abgesaugt, mit n-Octan nachgewaschen und getrocknet. Die Ausbeute beträgt 92 %.

Analog zu Beispiel 1 wurde 5,6-Dihydro-4-hydroxy-6-phenethyl-6-propyl-2H-pyran-2-on hergestellt, wobei Schritt a) in einem Mikroreaktor nach den in der folgenden Tabelle aufgeführten Bedingungen durchgeführt wurde:

| Beispiel Nr. | Temperatur [°C] | Volumenstrom I (1-Phenyl-3-hexanon) [ml/min] | Volumenstrom II (Acetessigsäure ester) [ml/min] | Ausbeute Ketoester [HPLC % Fläche] |
|---|---|---|---|---|
| 2 | -25 | 1 | 1 | 84,5 |
| 3 | -25 | 1 | 1,1 | 87,9 |
| 4 | -25 | 1 | 1,2 | 83,8 |
| 5 | -25 | 1 | 1,3 | 86,4 |
| 6 | -25 | 2 | 2,4 | 81,1 |
| 7 | -20 | 1 | 1,2 | 87,2 |
| 8 | -20 | 2 | 2,4 | 83,8 |
| 9 | -10 | 1 | 1,2 | 86,5 |
| 10 | -10 | 2 | 2,4 | 86,8 |
| 11 | 10 | 2 | 2,4 | 82,1 |

## Patentansprüche

1. Verfahren zur Herstellung einer Verbindung der allgemeinen Formel (I), 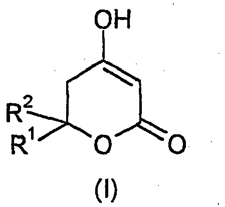 worin,
R¹ für einen C₁-C₈-Alkyl-, C₆-C₁₀-Aryl-C₁-C₄-alkyl-, oder C₃-C₈-Cycloalkyl-C₁-C₄-alkyl- Rest, und
R² für einen C₁-C₈-Alkyl-Rest steht,
durch Umsetzung a) eines Ketons der Formel (II) 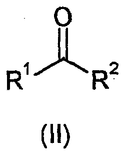 worin R¹ und R² die vorstehend genannte Bedeutung aufweist,
mit einem Acetessigester in Gegenwart einer starken Base
und
b) durch Zyklisierung der erhaltenen Verbindung der Formel (IV) 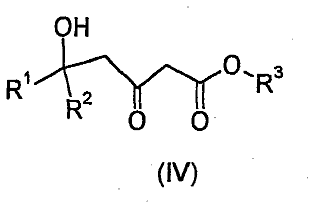 worin,
R³ für einen C₁-C₄-Alkyl-Rest steht,
mittels einer Base, **dadurch gekennzeichnet, dass** man eine Verbindung der Formel II kontinuierlich mit einem Acetessigester in Form seines Dianions in einem Mikroreaktor mischt und zur Reaktion bringt.

2. Verfahren nach Anspruch 1, **dadurch gekennzeichnet, dass** für Reaktionsschritt a) ein Mikroreaktor mit interdigitaler Kanalstruktur verwendet wird.

3. Verfahren nach Anspruch 1 oder 2, **dadurch gekennzeichnet, dass** ein die Verbindung der Formel (II) enthaltender Eduktstrom A und ein den Acetessigester in Form seines Dianions enthaltender Eduktstrom B kontinuierlich in dem Mischelement eines Mikroreaktors miteinander vermischt werden und das flüssige Reaktionsgemisch in eine Verweilzeit-Kapillare geführt wird.

4. Verfahren nach einem der Ansprüche 1 - 3 , **dadurch gekennzeichnet, dass** die Kapillare eine Länge von 0,1 bis 10 m und einen Durchmesser von 0,05 bis 5 mm besitzt.

5. Verfahren nach einem der Ansprüche 1 - 4, **dadurch gekennzeichnet, dass** in Schritt a) als Verbindung der Formel (II) 1-Phenyl-3-hexanon eingesetzt wird.

6. Verfahren nach einem der Ansprüche 1 - 5, **dadurch gekennzeichnet, dass** in Schritt a) der Acetessigester in Gegenwart von mindestens 2 Equivalenten einer starken Base ausgewählt aus der Gruppe bestehend aus Natriumhydrid, Butyllithium und Lithiumdialkylamid eingesetzt wird.

7. Verfahren nach einem der Ansprüche 1 - 6, **dadurch gekennzeichnet, dass** der Acetessigester zu der Verbindung der Formel (II) in einem molaren Verhältnis von 2:1 bis 1:2 eingesetzt wird.

8. Verfahren nach einem der Ansprüche 1 - 7, **dadurch gekennzeichnet, dass** die Reaktion in Schritt a) bei einer Temperatur von -78 bis +85 °C durchgeführt wird.

9. Verfahren nach einem der Ansprüche 1 - 9, **dadurch gekennzeichnet, dass** die Reaktion in Schritt a) bei einer Gesamtflussrate von 1,5 bis 5 ml/min durchgeführt wird.

10. Verfahren nach einem der Ansprüche 1 - 9, **dadurch gekennzeichnet, dass** die Flussrate der Verbindung der Formel (II) zu der Verbindung der Formel (III) in einem Verhältnis von 1:1 1 bis 1:2 steht.

11. Verfahren nach einem der Ansprüche 1 - 10, **dadurch gekennzeichnet, dass** die Reaktion in mehreren seriell oder parallel geschalteteten Mikroreaktoren durchgeführt wird.

12. Verwendung des nach einem Verfahren gemäß einem der Ansprüche 1 - 11 erhaltenen 5,6-Dihydro-4-hydroxy-6-phenethyl-6-propyl-2H-pyran-2-on zur Herstellung von Tipranavir.

## Claims

1. Process for preparing a compound of general formula (I), 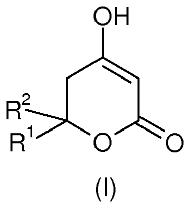 wherein
R¹ denotes a C₁-C₈-alkyl, C₆-C₁₀-aryl-C₁-C₄-alkyl, or C₃-C₈-cycloalkyl-C₁-C₄-alkyl group, and
R² denotes a C₁-C₈-alkyl group,
by reacting a) a ketone of formula (II) 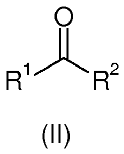 wherein R¹ and R² are as hereinbefore defined,
with an acetoacetate in the presence of a strong base
and
b) by cyclising the resulting compound of formula (IV) 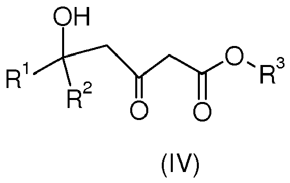 wherein
R³ denotes a C₁-C₄-alkyl group,
by means of a base, **characterised in that** a compound of formula II is continuously mixed and reacted with an acetoacetate in the form of its dianion in a microreactor.

2. Process according to claim 1, **characterised in that** a microreactor with an interdigital channel structure is used for reaction step a).

3. Process according to claim 1 or 2, **characterised in that** a current of educt A containing the compound of formula (II) and a current of educt B containing the acetoacetate in the form of its dianion are continuously mixed together in the mixing element of a microreactor and the liquid reaction mixture is passed into a holding capillary.

4. Process according to one of claims 1 - 3 , **characterised in that** the capillary is 0.1 to 10 m long and 0.05 to 5 mm in diameter.

5. Process according to one of claims 1 - 4, **characterised in that** 1-phenyl-3-hexanone is used as the compound of formula (II) in step a).

6. Process according to one of claims 1 - 5, **characterised in that** in step a) the acetoacetate is used in the presence of at least 2 equivalents of a strong base selected from among sodium hydride, butyllithium and lithium dialkylamide.

7. Process according to one of claims 1 - 6, **characterised in that** the acetoacetate is added to the compound of formula (II) in a molar ratio of 2:1 to 1:2.

8. Process according to one of claims 1 - 7, **characterised in that** the reaction in step a) is carried out at a temperature of -78 to +85°C.

9. Process according to one of claims 1 - 9, **characterised in that** the reaction in step a) is carried out at an overall flow rate of 1.5 to 5 ml/min.

10. Process according to one of claims 1 - 9, **characterised in that** the flow rate of the compound of formula (II) to the compound of formula (III) is in a ratio of 1:1 to 1:2.

11. Process according to one of claims 1 - 10, **characterised in that** the reaction is carried out in a plurality of microreactors connected in series or in parallel.

12. Use of the 5,6-dihydro-4-hydroxy-6-phenethyl-6-propyl-2H-pyran-2-one obtained by a process according to one of claims 1 - 11 for the preparation of tipranavir.

## Revendications

1. Procédé de production d'un composé de formule (I), 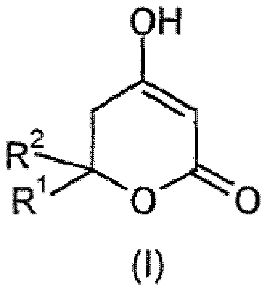 dans laquelle
R¹ désigne un radical alkyle en C₁ à C₈, aryle en C₆ à C₁₀-alkyle en C₁ à C₄ ou cycloalkyle en C₃ à C₈-alkyle en C₁ à C₄, et
R² désigne un radical alkyle en C₁ à C₈,
par la réaction a) d'une cétone de formule (II) 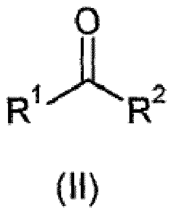 dans laquelle R¹ et R² a la signification mentionnée ci-dessus,
avec un ester acéto-acétique en présence d'une base forte
et
b) par cyclisation du composé de formule (IV) obtenu 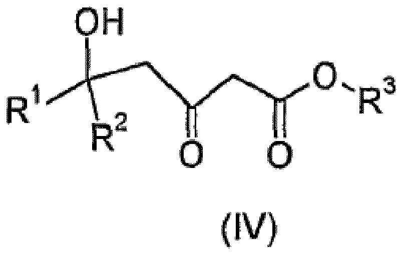
dans laquelle
R³ désigne un radical alkyle en C₁ à C₄,
au moyen d'une base, **caractérisé en ce que** l'on mélange et l'on fait réagir en continu un composé de formule II avec un ester acéto-acétique sous la forme de son dianion dans un microréacteur.

2. Procédé selon la revendication 1, **caractérisé en ce que**, pour l'étape réactionnelle a), on utilise un microréacteur présentant une structure canalaire interdigitée.

3. Procédé selon la revendication 1 ou 2, **caractérisé en ce que** l'on mélange ensemble en continu un courant de composant de départ A contenant le composé de formule (II) et un courant de composant de départ B contenant l'ester acéto-acétique sous la forme de son di-anion dans l'élément de mélange d'un microréacteur et le mélange réactionnel liquide est acheminé dans des capillaires à temps de séjour.

4. Procédé selon l'une des revendications 1 à 3, **caractérisé en ce que** les capillaires ont une longueur de 0,1 à 10 m et un diamètre de 0,05 à 5 mm.

5. Procédé selon l'une des revendications 1 à 4, **caractérisé en ce que**, à l'étape a) on utilise comme composé de formule (II), la 1-phényl-3-hexanone.

6. Procédé selon l'une des revendications 1 à 5, **caractérisé en ce que**, à l'étape a), l'ester acéto-acétique est utilisé en présence d'au moins 2 équivalents d'une base forte choisie dans le groupe comprenant l'hydrure de sodium, le butyllithium et le dialkylamide de lithium.

7. Procédé selon l'une des revendications 1 à 6, **caractérisé en ce que** l'ester acéto-acétique est utilisé pour le composé de formule (II) en un rapport molaire de 2:1 à 1:2.

8. Procédé selon l'une des revendications 1 à 7, **caractérisé en ce que** la réaction est réalisée à l'étape a) à une température de -78 à +85 °C.

9. Procédé selon l'une des revendications 1 à 9, **caractérisé en ce que** la réaction s'effectue à l'étape a) à un débit total de 1,5 à 5 ml/min.

10. Procédé selon l'une des revendications 1 à 9, **caractérisé en ce que** le débit du composé de formule (II) au composé de formule (III) est en un rapport de 1:1 à 1:2.

11. Procédé selon l'une des revendications 1 à 10, **caractérisé en ce que** la réaction s'effectue dans plusieurs microréacteurs montés en série ou en parallèle.

12. Utilisation de la 5,6-dihydro-4-hydroxy-6-phénéthyl-6-propyl-2H-pyran-2-one obtenue d'après un procédé selon l'une des revendications 1 à 11, pour la production de tipranavir.
